# EUROPEAN PATENT SPECIFICATION

(11) **EP 2 583 691 B1**
(45) Date of publication and mention of the grant of the patent: **16.01.2019**
(21) Application number: 11795835.5
(22) Date of filing: 17.06.2011
(51) Int. Cl.: A61K 47/10, A61K 9/127, A61K 31/07, A61K 31/7088, A61K 31/713, A61K 45/00, A61K 47/69, A61K 47/54, C12N 15/11, C12N 15/113

(54) **AGENT FOR TREATING RENAL FIBROSIS**
MITTEL ZUR BEHANDLUNG VON NIERENFIBROSE
AGENT POUR LE TRAITEMENT DE LA FIBROSE RÉNALE

(30) Priority: 17.06.2010 JP 2010138070
(43) Date of publication of application: 24.04.2013
(73) Proprietor: Nitto Denko Corporation, Ibaraki-shi Osaka 567-8680 (JP)
(72) Inventor: NIITSU, Yoshiro, Sapporo-shi Hokkaido 064-0823 (JP); KAJIWARA, Keiko, Ibaraki-shi Osaka 567-8680 (JP); TANAKA, Yasunobu, Ibaraki-shi Osaka 567-8680 (JP); MIYAZAKI, Miyono, Sapporo-shi Hokkaido 064-0914 (JP)
(74) Representative: Hoffmann Eitle
(86) International application number: PCT/JP2011/063910
(87) International publication number: WO 2011/158933

(56) References cited:
- EP-A1- 1 842 557
- WO-A1-2006/068232
- WO-A1-2010/014117
- WO-A2-2009/036368
- WO-A2-2009/036368
- WO-A2-2011/072082
- WO-A2-2012/170952
- JP-A- 2009 221 164
- JP-A- 2010 059 124
- KIDA YUJIRO ET AL.: 'Characterterization of vitamin A-storing cells in mouse fibrous kidneys using Cygb/STAP as a marker of activated stellate cells' ARCH HISTOL CYTOL vol. 70, no. 2, 2007, pages 95 - 106, XP055073691

### SEQUENCE LISTING

<110> NITTO DENKO CORPORATION
<120> AGENT FOR TREATING RENAL FIBROSIS
<130> PCT2453ND
<150> JP2010-138070
   <151> 2010-06-17
<160> 2
<170> PatentIn version 3.5
<210> 1
   <211> 21
   <212> DNA
   <213> Artificial
<220>
   <223> Hsp47-C sense strand
<400> 1
   ggacaggccu guacaacuat t 21
<210> 2
   <211> 21
   <212> DNA
   <213> Artificial
<220>
   <223> Hsp47-C antisense strand
<400> 2
   uaguuguaca ggccugucct t 21

## Description

### [Technical Field]

The present invention relates to a composition for treating diabetic nephritis utilizing a carrier targeted to extracellular matrix-producing cells in the kidney.

### [Background Art]

In recent years, due to great changes in lifestyle, mainly with respect to eating habits, the prevalence of lifestyle-related diseases such as high blood pressure and diabetes has increased, and accompanying this the risk of renal disease, and consequently renal failure, has been increasing.

The kidney is an important organ for maintaining homeostasis of the internal environment by means of excretion of waste products or regulation of bodily fluids/electrolytes/acid-base balance, etc. The kidney controls the concentrations of various compounds in the blood, such as those of hydrogen, sodium, potassium, and silicon, and excretes waste products in the form of urine. Any deterioration of renal function gives rise to the possibility of interference with the body's ability to sufficiently remove metabolites from the blood and also the possibility of destruction of the body's electrolyte balance. In its most serious form, deterioration or failure of renal function can be fatal.

Furthermore, other than the above-mentioned functions, the kidney has functions such as cell mediated immunity, endocrine secretion, and metabolism. The kidney as an endocrine organ carries out the production of erythropoietin, which regulates the production of red blood cells or 1,25-dihydroxyvitamin D3, which is an active form of vitamin D3, the secretion of renin and erythropoietin, which are blood pressure regulation factors, and the secretion of kinin, kallikrein, prostaglandin, etc.

Chronic renal failure means a state in which the above renal functions gradually deteriorate irreversibly and homeostasis of a living body cannot be maintained. It is known that chronic renal failure is caused by diabetic nephropathy, chronic glomerulonephritis, malignant nephrosclerosis, polycystic kidney disease, etc. All of the renal diseases are accompanied by fibrosis of the kidney and eventually lead to terminal renal failure. In particular, since chronic deterioration of renal function depends heavily on the progress of fibrosis of the kidney, it is thought that inhibiting the progress of fibrosis can result in suppression of the progress of chronic renal failure.

In general, renal fibrosis is accompanied by an inflammatory response due to endothelial cell dysfunction, and the extracellular matrix that is eventually produced in excess causes fibrosis. For example, in glomerulosclerosis, due to glomerular endothelial cell dysfunction, a cytokine such as a chemokine or a growth factor is secreted, and monocytes or macrophages migrate so as to make an inflammatory response progress. Subsequently, activation, proliferation, and transformation of mesangial cells occur, an excess amount of extracellular matrix is produced from extracellular matrix-producing cells such as mesangial cells, fibrosis occurs, and this leads to glomerulosclerosis.

In particular, diabetic nephropathy is the biggest factor leading to chronic renal failure, and one of the complications of diabetes. Diabetic nephropathy is characterized by hyperplasia and enlargement of the glomerular mesangium, and this is mainly due to an increase in accumulation of an ECM protein such as type I or type IV collagen, fibronectin, or laminin.

Once a serious degree of chronic renal failure occurs, recovery is impossible, and uremia will occur unless a treatment such as dialysis is carried out. As a therapy for chronic renal failure, a diet such as a low-protein diet or a salt-restricted diet, administration of an antihypertensive drug in order to alleviate the burden on the glomerulus, etc. is carried out.

Furthermore, for chronic renal failure, as necessary, a treatment for supplementing 1,25-dihydroxyvitamin D3 or erythropoietin, which are secreted by the kidney, or a treatment for appropriately maintaining blood pressure regulation, which is an important function of the kidney, is carried out.

As a chemotherapy, in order to suppress the progress of renal failure, an angiotensin converting enzyme inhibitor or an angiotensin II receptor antagonist is used. It is surmised that they have a renal protective effect per se in addition to suppressing the progress of renal failure by decreasing glomerular blood pressure. However, it is necessary to take care in administration since when the glomerular blood pressure is decreased too much the amount of glomerular blood flow instead decreases and prerenal renal failure occurs.

As the angiotensin converting enzyme inhibitor, there are captoril, enalapril, delapril, imidapril, quinapril, temocapril, perindopril erbumine, lisinopril, etc., and as the angiotensin II receptor antagonist there are losartan, valsartan, candesartan cilexetil, telmisartan, olmesartan medoxomil, irbesartan, etc.

Other than the above, in order to improve the symptoms of uremia and delay the start of dialysis a little, Kremezin is used as an adsorptive carbon that adsorbs harmful substances in the intestine. Furthermore, in order to prevent breathlessness, numbness in the limbs, arrhythmia, etc. due to an increase in potassium in the blood, calcium polystyrene sulfonate is used as an ion-exchange resin that adsorbs potassium in the intestine and is excreted in the feces.

When the concentration in serum of creatinine, which is a skeletal muscle-derived metabolite, exceeds 5 to 7 mg/dL, an artificial dialysis therapy such as peritoneal dialysis, hemofiltration, or blood dialysis, or a kidney transplant, can be considered. However, blood dialysis imposes a large burden on a person's life due to having to visit a hospital three times a week and being confined there for 4 to 5 hours per treatment, and with regard to a kidney transplant, since there are few kidney donors, among patients who desire one only a very small number can receive a kidney transplant. Furthermore, the average life expectancy of renal failure patients after starting dialysis is only about half that of the normal population.

In these circumstances, a great deal of research effort has been put into the development of an agent for treating renal fibrosis. As a result, it has been reported that, for example, a medicinal agent acting on the renin-angiotensin-aldosterone system such as an angiotensin converting enzyme inhibitor, an AT1 angiotensin II receptor antagonist, an aldosterone antagonist, or a renin inhibitor, a medicinal agent acting on the nitric oxide system such as an endothelin receptor antagonist, an α blocker, a β blocker, an immunosuppressive agent, an extracellular matrix metabolism inhibitor, a complement system inhibitor, a chemokine inhibitor, or a phosphodiesterase 5 inhibitor, and a medicinal agent such as an NFκB inhibitor, a Rho inhibitor, a p38 MAPK inhibitor, a PI3Kγ inhibitor, a vascular endothelium cell growth factor (VEGF) inhibitor, kallikrein, relaxin, an interleukin 1 receptor antagonist, bone morphogenetic factor 7 (BMP-7), antitumor necrosis factor α antibody (Anti-TNFa antibody), or anti-platelet-derived growth factor D antibody (Anti-PDGF-D antibody) have a certain degree of success with a renal fibrosis model animal or in a clinical trial (Non-Patent Document 1).

Furthermore, there have been many patent applications and, for example, patents relating to an angiotensin converting enzyme inhibitor (Patent Document 1), an angiotensin II receptor antagonist (Patent Document 2), a transforming growth factor β (TGF-β) inhibitor (Patent Document 3), a plasminogen activator inhibitor 1 (PAI-1) production inhibitor (Patent Document 4), a prostaglandin receptor 4 selective agonist (Patent Document 5), a type I collagen synthesis inhibitor (Patent Document 6), a chondroitin sulfate proteoglycan sulfotransferase inhibitor (Patent Document 7), a vitamin K epoxide reductase inhibitor (Patent Document 8), a glycation end product formation inhibitor (Patent Document 9), an A2A adenosine receptor 2A agonist (Patent Document 10), an endothelin receptor antagonist (Patent Document 11), a VEGF inhibitor (Patent Document 12), etc. have been applied for as drugs for treating renal fibrosis. However, none of these medicinal agents are satisfactory, and further development of agents for treating renal fibrosis is needed. Patent Documents 16 and 17 describe compositions that can include a carrier, targeting agent, and therapeutic agent. The therapeutic agent may have a therapeutic activity such as inhibiting fibrosis within a target organ or tissue or inhibiting the growth of a cancer cell. Patent Document 18 describes an astrocyte-specific drug carrier containing a retinoid derivative and/or a vitamin A analog as a constituent; a drug delivery method with the use of the same; a drug containing the same; and a therapeutic method with the use of the drug. By binding a drug carrier to a retinoid derivative such as vitamin A or a vitamin A analog or encapsulating the same in the drug carrier, a drug for therapeutic use can be delivered specifically to astrocytes. As a result, an astrocyte-related disease can be efficiently and effectively inhibited or prevented while minimizing side effects. As the drug inhibiting the activity or growth of astrocytes, for example, a siRNA against HSP47 which is a collagen-specific molecule chaperone may be encapsulated in the drug carrier. Thus, the secretion of type I to type IV collagens can be inhibited at the same time and, in its turn, fibrosis can be effectively inhibited. Patent Document 19 provides compositions, methods and kits for modulating expression of target genes, particularly heat shock protein 47 (hsp47) . The compositions, methods and kits may include nucleic acid molecules (for example, short interfering nucleic acid (siNA), short interfering RNA (siRNA), double-stranded RNA (dsRNA), micro-RNA (miRNA) or short hairpin RNA (shRNA)) that modulate a gene encoding hsp47, for example, the gene encoding human hsp47. The composition and methods disclosed therein may also be used in treating conditions and disorders associated with hsp47 such as liver fibrosis, pulmonary fibrosis, peritoneal fibrosis and kidney fibrosis.

### [Related Art Documents]

### [Patent Documents]

[Patent Document 1] US Pat. No. 5238924
[Patent Document 2] JP, A, 07-002667
[Patent Document 3] JP, A, 2004-043459
[Patent Document 4] JP, A, 2009-007258
[Patent Document 5] JP, A, 2001-233792
[Patent Document 6] JP, A (PCT) 2004-534760
[Patent Document 7] JP, A, 2009-292725
[Patent Document 8] JP, A, 2010-077101
[Patent Document 9] JP, A, 2009-029750
[Patent Document 10] JP, A (PCT) 2007-536241
[Patent Document 11] JP, A (PCT) 2006-519817
[Patent Document 12] JP, A, 2007-099641
[Patent Document 13] International Patent Application WO 2006/068232
[Patent Document 14] JP, A, 2009-221164
[Patent Document 15] JP, A, 2010-59124
[Patent Document 16] WO 2010/014117 A1
[Patent Document 17] WO 2009/036368 A2
[Patent Document 18] EP 1 842 557 A1
[Patent Document 19] WO 2011/072082 A2

### [Non-Patent Documents]

[Non-Patent Document 1] Nephrology, dialysis, transplantation 2007; 22(12): 3391-407

### [Summary of the Invention]

### [Problems to be Solved by the Invention]

It is an object of the present invention to provide an agent for treating diabetic nephritis utilizing a carrier that can specifically deliver a substance such as a drug to extracellular matrix-producing cells in the kidney.

### [Means for Solving the Problems]

The inventors have discovered, during an investigation into a novel agent for treating renal fibrosis, that renal fibrosis and especially diabetic nephritis could be treated effectively by administering a composition in which an extracellular matrix production inhibitor is carried by a carrier that includes a retinoid as an agent that promotes the delivery of a substance to extracellular matrix-producing cells in the kidney, thereby completing the invention.

It is known that a carrier that includes vitamin A can deliver a drug to stellate cells that store vitamin A (Patent Document 13), and that a composition in which siRNA for HSP47 is supported on the above carrier can improve hepatic fibrosis (Patent Document 13), pulmonary fibrosis (Patent Document 14), and myelofibrosis (Patent Document 15), but any relationship to renal fibrosis, renal interstitial tissue, or the mesangium is so far completely unknown.

That is, the present invention relates to the following.
(1) A pharmaceutical composition for use in treating diabetic nephritis, the composition comprising a carrier comprising a retinoid that promotes the delivery of a substance to extracellular matrix-producing cells in the kidney and a drug for controlling the activity or growth of extracellular matrix-producing cells in the kidney, wherein the carrier has the form of a liposome, and the molar ratio of retinoid and lipid contained in the liposome is 8:1 to 1:4.
(2) The pharmaceutical composition for use according to (1) above, wherein the retinoid includes retinol.
(3) The pharmaceutical composition for use according to any one of (1) or (2) above, wherein the drug for controlling the activity or growth of extracellular matrix-producing cells in the kidney is selected from the group consisting of an inhibitor of PAI-1 activity or production, a cell activity inhibitor, a growth inhibitor, an apoptosis inducer, and an RNAi molecule, ribozyme, antisense nucleic acid, or DNA/RNA chimeric polynucleotide that target at least one of the extracellular matrix constituent molecules or molecules involved in the production or secretion of the extracellular matrix constituent molecules or a vector expressing same.
(4) The pharmaceutical composition for use according to (1) or (2) above, wherein the drug for controlling the activity or growth of extracellular matrix-producing cells is an HSP47 inhibitor.
(5) The pharmaceutical composition for use according to any one of (1) to (4) above, wherein the drug and the carrier are mixed at a place of medical treatment or in its vicinity.

### [Effects of the Invention]

While the exact mode of action of the composition for treating diabetic nephritis of the present invention has not yet been completely clarified, it is believed that the retinoid functions as an agent that targets extracellular matrix-producing cells in the kidney such as fibroblasts or myofibroblasts, and delivers an active ingredient, namely a drug that controls the activity or growth of extracellular matrix-producing cells in the kidney to such cells, thereby exhibiting an effect against renal fibrosis and especially diabetic nephritis.

Therefore, since an active ingredient can be efficiently delivered to the site of action and, further, to target cells, by using the pharmaceutical composition of the present invention, the treatment, suppression of progression, and prevention of onset of diabetic nephritis, the treatment of which has been difficult to date, are made possible, and the present pharmaceutical composition thus contributes significantly to human medicine and veterinary medicine.

Moreover, the pharmaceutical composition of the present invention can be combined with any pharmaceutical agent (for example, an existing therapeutic agent for renal fibrosis) to increase its efficiency of action; it is therefore also advantageous for its broad range of application in terms of formulation, enabling the production of effective therapeutic agents to be facilitated.

### [Brief Description of Drawings]

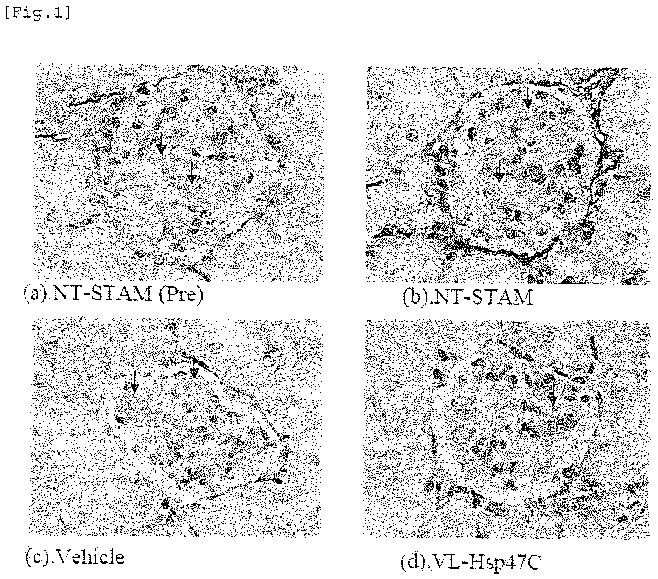

[FIG. 1] FIG. 1 is a representative microscopic image of the glomerulus by Sirius red staining of a section of the renal cortex of a mouse of each group. The images were taken at a magnification of 800× using an oil immersion lens.

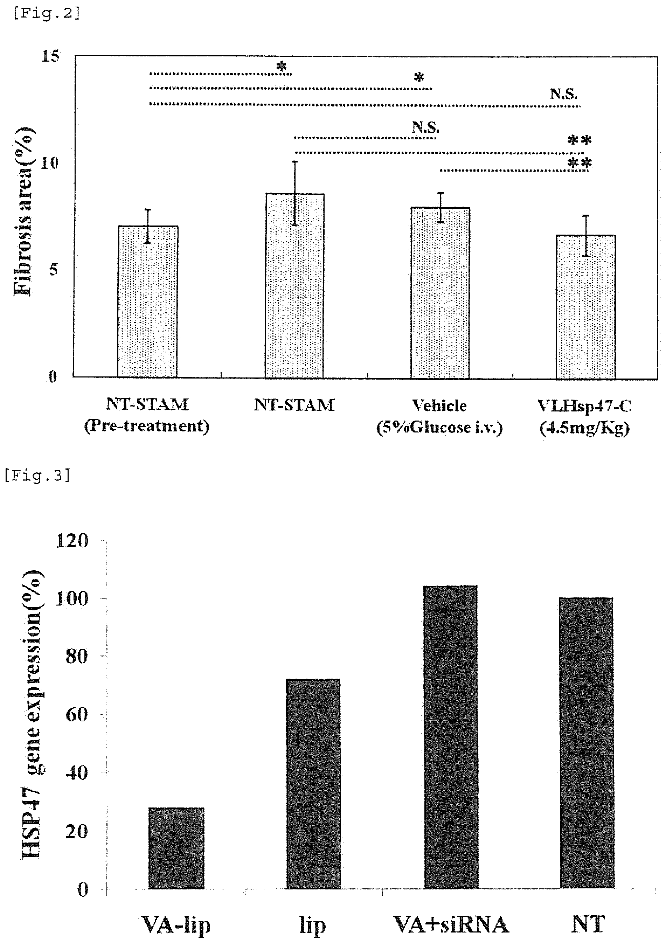

[FIG. 2] FIG. 2 is a graph showing the proportion of the fibrotic region of the renal cortex quantitatively determined by Sirius red staining. 20 fields of view were randomly taken in the renal cortex region per individual mouse, and the proportion of the fibrotic region (Fibrosis area (%)) was calculated (*P < 0.05, **P < 0.01).
[FIG. 3] FIG. 3 is a graph showing gene knockdown by siRNA-containing VA-binding liposome in mouse renal extracellular matrix-producing cells. The level of HSP47 gene expression in the extracellular matrix-producing cells collected from the mouse kidney was corrected with the expression level of GAPDH, which is an internal control gene, and the proportion of HSP47 gene expression(HSP47 gene expression (%)) was plotted while defining 'No treatment' (non-treated) as 100%. VA-lip denotes VA-liposome-siRNA Hsp47C, lip denotes liposome-siRNA Hsp47C, VA + siRNA denotes VA + siRNA Hsp47C, and NT denotes No treatment.

### [Modes for Carrying Out the Invention]

In the present invention, the extracellular matrix-producing cells in the kidney are not particularly limited as long as they are cells present in the kidney having the ability to produce extracellular matrix, and examples thereof present in the kidney include mesangial cells, tubulointerstitial cells, pericytes, fibroblasts, fibrocytes, which are precursor cells for fibroblasts, and myofibroblasts. The matrix-producing cells present in the kidney can include not only those derived from cells present in the kidney but also those derived from fibrocytes in circulating blood and those transformed from endothelial cells by endothelial-mesenchymal transdifferentiation. Myofibroblasts are characterized by the expression of α-SMA (alpha-smooth muscle actin). The myofibroblasts in the present invention are ones identified by for example immunostaining using detectably-labeled anti-α-SMA antibodies. Furthermore, since fibroblasts express vimentin, which is characteristic of mesenchymal cells, but do not express αSMA, they may be identified by double staining of vimentin and αSMA, etc. Moreover, the extracellular matrix-producing cells in the kidney may be obtained by treating renal tissue with collagenase and protease and then carrying out isolation by density-gradient centrifugation (e.g. in Nycodenz® having a final concentration of 8%).

The retinoid employed in the present invention functions as a targeting agent (targeting agent) to extracellular matrix-producing cells in the kidney, and promotes the specific delivery of a substance to these cells. The mechanism of the promotion of substance delivery by the retinoid has not yet been completely clarified; however, for example, it is thought that a retinoid that has specifically bound to a retinol-binding protein (RBP) is taken into an extracellular matrix-producing cell in the kidney through a certain receptor present on the surface of said cell.

A retinoid is a member of a class of compounds having a skeleton in which four isoprenoid units are bonded in a head-to-tail manner (see G. P. Moss, 'Biochemical Nomenclature and Related Documents', 2nd Ed. Portland Press, pp. 247-251 (1992)). Vitamin A is a generic descriptor for a retinoid that qualitatively shows the biological activity of retinol. The retinoid that can be used in the present invention is not particularly limited, and examples thereof include retinol (including all-trans-retinol), retinal, retinoic acid (including tretinoin), retinoid derivatives such as an ester of retinol and a fatty acid, an ester of an aliphatic alcohol and retinoic acid, etretinate, isotretinoin, adapalene, acitretine, tazarotene, and retinyl palmitate, and vitamin A analogues such as fenretinide (4-HPR) and bexarotene.

Of these, retinol, retinal, retinoic acid, an ester of retinol and a fatty acid (such as retinyl acetate, retinyl palmitate, retinyl stearate, and retinyl laurate) and an ester of an aliphatic alcohol and retinoic acid (such as ethyl retinoate) are preferable from the viewpoint of efficiency of specific delivery of a substance to extracellular matrix-producing cells in the kidney.

All retinoid isomers including cis-trans isomers are included in the scope of the present invention. The retinoid may be substituted with one or more substituents.

The carrier that is present in the pharmaceutical composition of the present invention may be constituted by binding the retinoid to a carrier constituent component other than the retinoid, or by enclosing it therein. Therefore, the carrier employed in the present invention includes a carrier constituent component other than the retinoid.

Examples of such a component include a lipid, for example, a phospholipid such as glycerophospholipid, a sphingolipid such as sphingomyelin, a sterol such as cholesterol, a vegetable oil such as soybean oil or poppy seed oil, a mineral oil, a lecithin such as egg-yolk lecithin, and a polymer, but the examples are not limited thereto. Among them, those that can form a liposome, for example, a natural phospholipid such as lecithin, a semisynthetic phospholipid such as dimyristoylphosphatidylcholine (DMPC), dipalmitoylphosphatidylcholine (DPPC), or distearoylphosphatidylcholine (DSPC), dioleylphosphatidylethanolamine (DOPE), dilauroylphosphatidylcholine (DLPC), and cholesterol are suitable.

A particularly preferred component is a component that can avoid capture by the reticuloendothelial system, examples thereof including cationic lipids such as *N*-(α-trimethylammonioacetyl)-didodecyl-D-glutamate chloride (TMAG), *N,N',N'',N'''*-tetramethyl-*N,N',N'',N'''*-tetrapalmitylspermine (TMTPS), 2,3-dioleyloxy-*N*-[2-(sperminecarboxamido)ethyl]-*N,N*-dimethyl-1 -propanaminium trifluoroacetate (DOSPA), *N*-[1-(2,3-dioleyloxy)propyl]-*N,N,N*-trimethylammonium chloride (DOTMA), dioctadecyldimethylammonium chloride (DODAC), didodecylammonium bromide (DDAB), 1,2-dioleyloxy-3-trimethylammoniopropane (DOTAP), 3β-[*N*-(*N',N'*-dimethylaminoethane)carbamoyl]cholesterol (DC-Chol), 1,2-dimyristoyloxypropyl-3-dimethylhydroxyethylammonium (DMRIE), and *O,O'*-ditetradecanoyl-*N*-(α-trimethylammonioacetyl)diethanolamin e chloride (DC-6-14).

The carrier employed in the present invention has the form of a liposome,.

In the present invention, the binding of the retinoid to the carrier or the enclosing of it therein is also made possible by binding the retinoid to or enclosing it in a carrier constituent other than the retinoid by a chemical and/or physical method. Alternatively, the retinoid can be bound to or enclosed in the carrier by mixing the retinoid and the carrier constituents other than the retinoid during the preparation of the carrier. In the present invention, the amount of the retinoid in the carrier may be for example 0.01 to 1000 nmol/µL, and is preferably 0.1 to 100 nmol/µL. The retinoid may be bound to or enclosed in the carrier before supporting a drug on the carrier; or the carrier, retinoid and drug may be mixed simultaneously; or the retinoid may be admixed with the carrier already supporting the drug, etc. This may be done by a process for producing a formulation specific to extracellular matrix-producing cells in the kidney, the process including a step of binding a retinoid to a liposomal formulation such as DaunoXome®, Doxil, Caelyx®, or Myocet®.

The carrier employed in the present invention is a liposome. In the present invention, a liposomal form is advantageously used from the viewpoint of high delivery efficiency, wide selection of substances to be delivered, and ease of formulation, etc., and a cationic liposome containing a cationic lipid is particularly preferable. The molar ratio of the retinoid to other constituents of the liposome is 8:1 to 1:4, and preferably 4:1 to 1:2, from the viewpoint of the efficiency of binding the retinoid to the carrier or enclosing it therein.

The employed in the pharmaceutical composition of the present invention contains a substance to be transported within its interior, it may be attached to the exterior of a substance to be transported, or it may be mixed with a substance to be transported, as long as it contains a retinoid in a form such that the retinoid is able to function as a targeting agent. 'Function as a targeting agent' herein means that the carrier that includes a retinoid reaches and/or is taken up by the target cells, i.e., extracellular matrix-producing cells in the kidney, more rapidly and/or in a larger quantity than with a carrier not including the retinoid, and this may easily be confirmed by, for example, adding a labeled carrier or label-containing carrier to a culture of target cells and analyzing the distribution of the label after a predetermined period of time. Structurally, this requirement can be satisfied, for example, if a retinoid is at least partially exposed to the exterior of the formulation containing the carrier at the latest by the time it reaches the target cells. The 'formulation' referred to here is a concept that includes the composition of the present invention, which is described later, and that further has a form. Whether or not the retinoid is exposed at the exterior of a formulation can be evaluated by contacting the formulation with a substance that specifically binds to a retinoid, such as for example a retinol-binding protein (RBP), and examining its binding to the formulation.

Exposing a retinoid at least partially to the exterior of the formulation at the latest by the time it reaches the target cells may be achieved for example by adjusting the compounding ratio of the retinoid and carrier constituent components other than the retinoid. Furthermore, as the carrier has the form of a liposome, when for example forming a complex from a retinoid and a carrier constituent component other than the retinoid, a method in which first a lipid structure formed from the carrier constituent component other than the retinoid is diluted in an aqueous solution, and this is then contacted and mixed with the retinoid, etc. may be used. In this case, the retinoid may be in a state in which it is dissolved in a solvent, for example, an organic solvent such as DMSO. The lipid structure referred to here means a three-dimensional liposome structure containing a lipid as a constituent component. Application to another drug carrier of the same targeting agent as one used with a liposome in targeting is described in for example Zhao and Lee, Adv Drug Deliv Rev. 2004; 56(8): 1193-204, Temming et al., Drug Resist Updat. 2005; 8(6): 381-402.

The lipid structure may be stabilized by for example adjusting the osmotic pressure by the use of an osmotic pressure-adjusting agent such as a salt, a saccharide such as sucrose, glucose, or maltose, or a polyhydric alcohol such as glycerol or propylene glycol, and preferably sucrose or glucose. Furthermore, the pH may be adjusted by adding an appropriate amount of an adjusting agent such as a salt or a buffer. It is therefore possible to carry out production, storage, etc. of a lipid structure in a medium containing the above substances. In this case, the concentration of the osmotic pressure-adjusting agent is preferably adjusted so as to be isotonic with blood. For example, in the case of sucrose the concentration thereof in a medium is, although not limited to, 3 to 15 wt%, preferably 5 to 12 wt%, more preferably 8 to 10 wt%, and particularly 9 wt%, and in the case of glucose the concentration thereof in a medium is, although not limited to, 1 to 10 wt%, preferably 3 to 8 wt%, more preferably 4 to 6 wt%, and particularly 5 wt%.

Also disclosed is a process for producing a carrier for delivering a substance to extracellular matrix-producing cells in the kidney, the process including a step of formulating a retinoid as a targeting agent to extracellular matrix-producing cells in the kidney. The method of formulating the retinoid is not particularly limited as long as, in the carrier in which it is formulated, the retinoid can function as an agent targeting extracellular matrix-producing cells in the kidney, and for example various methods described in the present specification may be used. Therefore, formulation of the retinoid may be carried out by binding the retinoid to or enclosing it in another constituent component of the carrier by a chemical and/or physical method or by mixing the retinoid with another carrier constituent component when preparing the carrier. The amount of retinoid formulated, etc. is as described above.

The substance or object that is delivered by the carrier described herein is not particularly limited, and it preferably has a size such that it can physically move within the body of an organism from the site of administration to the site of a lesion where the target cells are present. Therefore, the carrier described herein can transport not only a substance such as an atom, a molecule, a compound, a protein, or a nucleic acid, but also an object such as a vector, a virus particle, a cell, a drug-releasing system that includes one or more elements, or a micromachine. The substance or object preferably has the property of having some effect on the target cells, and examples include those labeling the target cells or controlling (e.g. increasing or suppressing) the activity or growth of the target cells.

In the present invention, it is 'a drug for controlling the activity or growth of extracellular matrix-producing cells in the kidney' that is delivered by the carrier. The activity of the extracellular matrix-producing cells in the kidney herein refers to various activities such as secretion, uptake, or migration exhibited by extracellular matrix-producing cells in the kidney, and in the present invention, in particular, among these, it typically means an activity involved in the onset, progression, and/or recurrence of renal fibrosis and especially diabetic nephritis. Examples of such activity include, but are not limited to, the production/secretion of a biologically active substance such as PAI-1, and of an extracellular matrix component such as collagen, proteoglycan, tenascin, fibronectin, thrombospondin, osteopontin, osteonectin, or elastin, and the suppression of decomposition activity of these extracellular matrix components.

Therefore, the drug for controlling the activity or growth of extracellular matrix-producing cells in the kidney referred to in the present specification may be any drug that directly or indirectly suppresses the physical, chemical, and/or physiological actions, etc. of said cells related to the onset, progression and/or recurrence of renal fibrosis, and examples thereof include, but are not limited to, a drug that inhibits the activity or production of the biologically active substance above, antibodies and antibody fragments that neutralize the biologically active substance above, a substance that suppresses the expression of the biologically active substance above, such as an RNAi molecule (e.g. siRNA, shRNA, ddRNA, miRNA, piRNA, rasiRNA), a ribozyme, an antisense nucleic acid (including RNA, DNA, PNA, or a composite thereof), or a substance having a dominant negative effect such as a dominant negative mutant, or a vector expressing these, or a drug that inhibits the production and secretion of the extracellular matrix component above, for example, a substance that suppresses the expression of the extracellular matrix component, such as an RNAi molecule (e.g. siRNA, shRNA, ddRNA, miRNA, piRNA, rasiRNA), a ribozyme, an antisense nucleic acid (including RNA, DNA, PNA, or a composite thereof), or a substance having a dominant negative effect such as a dominant negative mutant, or a vector expressing these, an inhibitor of cell activity such as a sodium channel blocker, a cell-growth inhibitor such as an alkylating agent (such as ifosfamide, nimustine, cyclophosphamide, dacarbazine, melphalan, or ranimustine), an antitumor antibiotic (such as idarubicin, epirubicin, daunorubicin, doxorubicin, pirarubicin, bleomycin, peplomycin, mitoxantrone, or mitomycin C), an antimetabolite (such as gemcitabine, enocitabine, cytarabine, tegafur/uracil, a tegafur/gimeracil/oteracil potassium mixture, doxifluridine, hydroxycarbamide, fluorouracil, methotrexate, or mercaptopurine), an alkaloid such as etoposide, irinotecan, vinorelbine, docetaxel, paclitaxel, vincristine, vindesine, or vinblastine, and a platinum complex such as carboplatin, cisplatin, or nedaplatin, as well as an apoptosis inducer such as cyclosporine.

Examples of the drug for inhibiting the production/secretion of an extracellular matrix component include, but are not limited to, HSP47, which is a collagen-specific molecular chaperone essential for intracellular transport and molecule maturation, which are common to the synthetic processes of various types of collagen.

Furthermore, the 'drug for controlling the activity or growth of extracellular matrix-producing cells in the kidney' in the present invention may be any drug that directly or indirectly promotes the physical, chemical and/or physiological actions of extracellular matrix-producing cells in the kidney directly or indirectly related to the suppression of onset, progression and/or recurrence of renal fibrosis, for example, the production/secretion of MMP (including MMP1, MMP2), a plasminogen activator (PA). Examples of such a drug include, but are not limited to, an activator or an expression enhancer for these substances. The pharmaceutical composition of the present invention may deliver one or more types of the above-mentioned drugs.

The siRNA (small interfering RNA) that can be used in the present invention includes, in addition to siRNA in the strict sense, duplex RNAs and modified forms thereof such as miRNA (micro RNA), shRNA (short hairpin RNA), piRNA(Piwi-interacting RNA), and rasiRNA (repeat associated siRNA). An siRNA as a functional small RNA in a wide sense and a vector expressing the siRNA may be used for example in accordance with instructions in a standard text (Experimental Medicine Special Edition, Revised RNAi Experimental Protocol 2004, Yodosha, RNAi Experimental Frequently Asked Questions 2006, Yodosha).

Design of these siRNAs may be carried out appropriately by a person skilled in the art in accordance with instructions in a standard text (Experimental Medicine Special Edition, Revised RNAi Experimental Protocol 2004, Yodosha, RNAi Experimental Frequently Asked Questions 2006, Yodosha) by reference to a messenger RNA sequence of a target gene and a known siRNA sequence.

The substance to be delivered by the pharmaceutical composition of the present invention may be, but is not limited to, a drug for suppressing the onset, progression, and/or recurrence of renal fibrosis other than the above-mentioned drugs, and examples thereof include, but are not limited to, a medicinal agent acting on the nitric oxide system such as an angiotensin converting enzyme inhibitor, an AT1 angiotensin II receptor antagonist, an aldosterone antagonist, an endothelin receptor antagonist, an α blocker, a β blocker, an immunosuppressive agent, an extracellular matrix metabolism inhibitor, a complement system inhibitor, a chemokine inhibitor, a TGF-β inhibitor, or a phosphodiesterase 5 inhibitor, an NFκB inhibitor, a Rho inhibitor, a p38 MAPK inhibitor, a PI3Kγ inhibitor, kallikrein, an interleukin 1 receptor antagonist, BMP-7, an antitumor necrosis factor α antibody, an anti-platelet-derived growth factor D antibody, a plasminogen activator inhibitor-1 production inhibitor, a prostaglandin receptor 4 selective agonist, a type I collagen synthesis inhibitor, a chondroitin sulfate proteoglycan sulfotransferase inhibitor, a vitamin K epoxide reductase inhibitor, a glycation end product formation inhibitor, an A2A adenosine receptor 2A agonist, an endothelin receptor antagonist, a VEGF inhibitor, an activator or an expression enhancer for ADAM (a disintegrin and metalloprotease domain) protease, an activator or an expression enhancer for ADAMTS (a disintegrin and metalloprotease with thrombospondin motifs) protease, and relaxin or an expression enhancer therefor provided the pharmaceutical composition of the present invention contains at least one drug for controlling the activity or growth of extracellular matrix-producing cells in the kidney. In the present specification, examples of the expression enhancer include, but are not limited to, a gene therapy agent such as a vector containing a nucleic acid coding for a protein that is a target for expression enhancement. These drugs may be used in combination with the composition of the present invention, which is described later. 'Used in combination' includes substantially simultaneous administration of the composition of the present invention and the above-mentioned drug and administration thereof with spaced timing within the same treatment period. In the case of the latter, the composition of the present invention may be administered either before or after the drug.

In one embodiment of the present invention, as the drug for controlling the activity or growth of extracellular matrix-producing cells in the kidney, an HSP47 inhibitor, for example, an siRNA corresponding to each thereof can be cited.

The substance or object delivered by the carrier contained in the pharmaceutical composition of the present invention may or may not be labeled. Labeling enables monitoring of the success or failure of delivery to target cells, or the increase and decrease of target cells, etc., and is particularly useful not only at the testing/research level but also at the clinical level. A label may be selected from any label known to a person skilled in the art such as, for example, any radioisotope, magnetic material, substance that binds to a labeled substance (e.g. an antibody), fluorescent substance, fluorophore, chemiluminescent substance, enzyme. A label may be affixed to a carrier constituent component or may be supported on a carrier as an independent substance to be delivered.

In the present invention, 'for extracellular matrix-producing cells in the kidney' or 'for delivery to extracellular matrix-producing cells in the kidney' means that it is suitable for use for extracellular matrix-producing cells in the kidney as target cells, and this includes, for example, it being possible to deliver a substance to these cells, more rapidly, efficiently, and/or in a larger quantity than to other cells, for example, normal cells. For example, the carrier of the present invention can deliver a substance to extracellular matrix-producing cells in the kidney at a rate and/or efficiency of 1.1 times or more, 1.2 times or more, 1.3 times or more, 1.5 times or more, 2 times or more, or even 3 times or more compared with other cells.

The present invention thus relates to a pharmaceutical composition for treating diabetic nephritis that contains a drug for controlling the activity or growth of extracellular matrix-producing cells in the kidney, the composition including the above-mentioned carrier in addition to the above-mentioned drug for controlling the activity or growth of extracellular matrix-producing cells in the kidney, and further described is a use of the carrier in the production of said composition.

The term renal fibrosis includes any interstitial nephritis, for example, streptococcal nephritis, staphylococcal nephritis, pneumococcal nephritis, varicella, hepatitis B, hepatitis C, viral nephritis associated with HIV, nephritis due to a parasitic infection such as malaria, infectious interstitial nephritis associated with fungal nephritis, mycoplasma nephritis, systemic lupus erythematosus (lupus nephritis), systemic scleroderma (kidney collagen disease), interstitial nephritis associated with a collagen disease such as Sjogren syndrome, nephritis associated with a blood vessel immune disease such as purpura nephritis, polyarteritis, or rapidly progressive glomerulonephritis, interstitial nephritis associated with radiation exposure, drug-induced interstitial nephritis due to a gold drug, an NSAID, penicillamine, an anticancer agent such as bleomycin, an antibiotic, Paraquat, allergic nephritis due to insect bite, pollen, an *Anacardiaceae* family plant, amyloidosis nephritis, diabetic nephropathy, chronic glomerulonephritis, nephritis associated with malignant nephrosclerosis, a polycystic kidney disease, tubulointerstitial nephritis, nephritis associated with gestational toxicosis or a cancer, membranoproliferative glomerulonephritis, IgA nephropathy, mixed cryoglobulinemic nephritis, Goodpasture's syndrome nephritis, Wegener's granulomatous nephritis, and a chronic form of interstitial nephritis caused by idiopathic interstitial nephritis such as acute interstitial nephritis. In the present invention diabetic nephritis is to be treated.

In the pharmaceutical composition of the present invention, as long as the retinoid contained in the carrier is present in a mode such that it functions as a targeting agent, the carrier may contain a substance to be delivered within its interior, it may be attached to the exterior of a substance to be delivered, or may be mixed with a substance to be delivered. Therefore, depending on the administration route and the manner in which the drug is released, etc., the composition may be covered with an appropriate material such as, for example, an enteric coating or a timed-disintegration material, or may be incorporated into an appropriate drug release system. Furthermore, the composition of the present invention may be in the form of a complex of an active ingredient and a retinoid-binding liposome, that is, a lipoplex.

The composition of the present invention is to be used as a medicine (that is, a pharmaceutical composition) and may be administered via various routes including both oral and parenteral routes, and examples thereof include, but are not limited to, oral, intravenous, intramuscular, subcutaneous, local, intrapulmonary, tracheobronchial, intratracheal, intrabronchial, nasal, intrarectal, intraarterial, intraportal, intraventricular, intramedullary, intra-lymph node, intra-lymphatic, intracerebral, intrathecal, intracerebroventricular, transmucosal, percutaneous, intranasal, intraperitoneal, and intrauterine routes, and it may be formulated into a dosage form suitable for each administration route. Such a dosage form and formulation method may be selected as appropriate from any known dosage form and method (see e.g. Hyojun Yakuzaigaku (Standard Pharmaceutics), Ed. by Yoshiteru Watanabe et al., Nankodo, 2003).

Examples of dosage forms suitable for oral administration include, but are not limited to, powder, granule, tablet, capsule, liquid, suspension, emulsion, gel, and syrup, and examples of dosage forms suitable for parenteral administration include injections such as an injectable solution, an injectable suspension, an injectable emulsion, and an injection to be prepared at the time of use. Formulations for parenteral administration may be in a form such as an aqueous or nonaqueous isotonic sterile solution or suspension.

The present disclosure also provides a process for producing a pharmaceutical composition for treating renal fibrosis, the process including a step of formulating a retinoid as a targeting agent for extracellular matrix-producing cells in the kidney and a drug for controlling the activity or growth of extracellular matrix-producing cells in the kidney as an active ingredient. The method for formulating the retinoid is not particularly limited as long as the retinoid can function as a targeting agent for extracellular matrix-producing cells in the kidney in the composition in which it is formulated, and for example various methods described in the present specification may be used. Furthermore, the method for formulating the active ingredient is not particularly limited as long as the active ingredient can exhibit a predetermined effect, and any known method may be used. Formulation of the active ingredient may be carried out at the same time as formulation of the retinoid or may be carried out before or after formulating the retinoid. For example, when the composition contains a carrier constituent component other than the retinoid, formulation of the active ingredient may be carried out by mixing the active ingredient with a carrier in which the retinoid has already been formulated as the targeting agent, it may be carried out by mixing the retinoid, a carrier constituent component other than the retinoid, and the active ingredient at the same time, or it may be carried out by formulating the active ingredient with a carrier constituent component other than the retinoid and then mixing this with the retinoid.

The amount of retinoid formulated, etc. is as described above with respect to the carrier as employed in the present invention. Furthermore, the amount of active ingredient is an amount that, when administered as the composition, can suppress the onset or recurrence of renal fibrosis and especially diabetic nephritis, improve the clinical condition, alleviate its symptoms, or delay or bring to a halt its progression, and preferably may be an amount that can prevent the onset or recurrence of renal fibrosis or cure it. It is also preferably an amount that does not cause an adverse effect that exceeds the benefit from administration. Such an amount may be known or be appropriately determined by an in vitro test using cultured cells or by a test in a model animal such as a mouse, rat, dog or pig, and such test methods are well known to a person skilled in the art. Examples of a model animal with renal fibrosis include one described in JP, A, 2009-178143. The amount of active ingredient formulated can vary according to the form of administration of the composition. For example, when a plurality of units of the composition are used in one administration, the amount of active ingredient formulated in one unit of the composition may be one obtained by dividing the amount of active ingredient required for one administration by the number of units. Such adjustment of the amount formulated may be carried out appropriately by a person skilled in the art.

The pharmaceutical composition of the present invention may be provided in any form, but from the viewpoint of stability on storage, it may preferably be provided in a form that can be prepared at the time of use, for example in a form such that it can be prepared at a place of medical treatment or in the vicinity thereof by a doctor and/or pharmacist, nurse or other paramedic. In this case, the pharmaceutical composition of the present invention is provided as one or more containers containing at least one constituent essential therefor, and it is prepared prior to use, for example, within 24 hours prior to use, preferably within 3 hours prior to use, and more preferably, immediately prior to use. When carrying out preparation, a reagent, a solvent, preparation equipment, etc. that are normally available at the place of preparation may be used as appropriate.

Accordingly, the present disclosure also provides a kit for preparing a carrier or composition, the kit including one or more containers that contain singly or in combination a retinoid, and/or a substance to be delivered, and/or a carrier constituent substance other than the retinoid, as well as to a constituent that is necessary for the carrier or composition provided in the form of such a kit. The kit described herein may contain, in addition to the above, instructions such as for example a written explanation or an electronic recording medium such as a CD or DVD regarding methods for preparing or administering the carrier and composition of the present invention. Furthermore, the kit described herein may contain all of the constituents for completing the pharmaceutical composition of the present invention, but need not necessarily contain all of the constituents. Accordingly, the kit described herein need not contain a reagent or solvent that is normally available at a place of medical treatment, an experimental facility, etc., such as, for example, sterile water, physiological saline, or glucose solution.

The present disclosure further provides a method for controlling the activity or growth of extracellular matrix-producing cells in the kidney or for treating renal fibrosis, the method including administering an effective amount of the above composition to a subject in need thereof. Here, the effective amount in for example a method for treating renal fibrosis is an amount that suppresses the onset or recurrence of renal fibrosis, improves the clinical condition, alleviates its symptoms, or delays or brings to a halt its progression, and is preferably an amount that prevents the onset or recurrence of renal fibrosis or cures it. It is also preferably an amount that does not cause an adverse effect that exceeds the benefit from administration. Such an amount may be appropriately determined by an in vitro test using cultured cells or by a test in a model animal such as a mouse, rat, dog, or pig, and such test methods are well known to a person skilled in the art. Moreover, the dose of the retinoid contained in the carrier and the dose of the drug used in the method described herein are known to a person skilled in the art, or may be appropriately determined by the above-mentioned test, etc. Examples of a model animal with renal fibrosis include one described in JP, A, 2009-178143.

The specific dose of the composition administered in the method described herein may be determined taking into account various conditions with respect to the subject in need of the treatment, such as the severity of symptoms, the general health condition of the subject, the age, body weight, and gender of the subject, diet, the administration route, the timing and frequency of administration, concurrent medication, responsiveness to the treatment, compliance with the treatment, etc.

The route of administration includes various routes including both oral and parenteral routes such as, for example, oral, intravenous, intramuscular, subcutaneous, local, intrapulmonary, tracheobronchial, intratracheal, intrabronchial, nasal, intrarectal, intraarterial, intraportal, intraventricular, intramedullary, intra-lymph node, intra-lymphatic, intracerebral, intrathecal, intracerebroventricular, transmucosal, percutaneous, intranasal, intraperitoneal, and intrauterine routes.

The frequency of administration varies depending on the properties of the composition to be used and the aforementioned conditions of the subject, and may be, for example, a plurality of times per day (more specifically, 2, 3, 4, 5, or more times per day), once a day, every few days (more specifically, every 2, 3, 4, 5, 6, or 7 days, etc.), a few times per week (e.g. 2, 3, 4 times, etc. per week), once a week, or every few weeks (more specifically, every 2, 3, 4 weeks, etc.).

In the method described herein, the term 'subject' means any living individual, preferably an animal, more preferably a mammal, and yet more preferably a human individual. The subject may be healthy or affected by some disorder, and when treatment of renal fibrosis is intended, it typically means a subject affected by diabetic nephritis or renal fibrosis or at risk of being affected thereby. When prevention of renal fibrosis is intended, for example, typical examples include, but are not limited to, a subject affected by diabetic nephritis, in particular diabetic nephritis due to type II diabetes.

Furthermore, the term 'treatment' includes all types of medically acceptable prophylactic and/or therapeutic intervention for the purpose of the cure, temporary remission, or prevention of a disorder. For example, the term 'treatment' includes medically acceptable intervention for various purposes, including delaying or halting the progression of renal fibrosis, the regression or disappearance of a lesion, and the prevention of onset and prevention of recurrence of renal fibrosis.

The present disclosure also provides a method utilizing the above carrier for delivering a drug to extracellular matrix-producing cells in the kidney. This method includes, but is not limited to, for example, a step of supporting a substance to be delivered on the carrier, and a step of administering or adding the carrier supporting the substance to be delivered to an organism or a medium, for example a culture medium, which contains extracellular matrix-producing cells in the kidney. These steps may appropriately be achieved according to any known method or a method described in the present specification. The delivery method may be combined with another delivery method, for example, another delivery method for targeting the kidney. Moreover, the method includes an embodiment performed in vitro and an embodiment in which extracellular matrix-producing cells in the kidney inside the body are targeted.

### [Examples]

The present invention is explained below in further detail by reference to the Examples, but they are only Examples and do not at all limit the present invention as defined by the appended claims.

### Example 1 Preparation of siRNA-containing VA-binding liposome

As the siRNA, one having the following sequence was used. Sequence name: Hsp47-C
5'-GGACAGGCCUGUACAACUA-dTdT-3'(sense, SEQ ID NO: 1)
5'-UAGUUGUACAGGCCUGUCC-dTdT-3'(antisense, SEQ ID NO: 2)

As solutions prior to mixing, 10 mM vitamin A (retinol, Sigma; hereinafter also called VA, dissolved in dimethyl sulfoxide), 1 mM Lipotrust SR (Hokkaido System Science Co., Ltd.; hereinafter also called a liposome or a liposome-constituting lipid, dissolved in nuclease-free water), and 10 µg/µL siRNA (Hsp47-C was dissolved in nuclease-free water) were prepared. Subsequently, VA dissolved in dimethyl sulfoxide was added to the Lipotrust SR dissolved in nuclease-free water prepared above at a ratio of 1:1 (mol/mol), and the mixture was stirred by means of a vortex for 15 seconds and then allowed to stand at room temperature for 5 minutes in a light-shielded state, thus forming a complex. This complex was mixed with siRNA, thus giving a VA Liposome-siRNA Hsp47C administration solution. This administration solution contained, per 100 µL, 75 nmol of VA, 75 nmol of liposome-constituting lipid, and 112.5 µg of siRNA, this corresponding to 3.00 µmol/kg of body weight of VA, 3.00 µmol/kg of body weight of the liposome-constituting lipid, and siRNA 4.5 mg/kg of body weight of siRNA. VA was exposed on the liposome surface.

### Example 2 Examination of therapeutic effect in renal fibrosis model mouse

### (1) Preparation of renal fibrosis model animal

Preparation of renal fibrosis model mice was commissioned from Stelic Institute & Co. Specifically, 2 day old C57BL6J/JcL male mice (CLEA Japan, Inc.) after birth were given an *N*-acetyl-β-D-glucosaminidase inhibitor, reared by feeding with CE-2 feed (CLEA Japan, Inc.) and sterile water up to 4 weeks old, weaned when they reached an age of 4 weeks, and then reared by feeding with High Fat Diet 32 (CLEA Japan, Inc.), which has a higher crude fat content than that of normal diet, and sterile water up to 12 weeks old, thus preparing STAM mice. It is known that these model mice will be affected by diabetic nephritis (see JP, A, 2009-178143), and renal fibrosis due to diabetic nephritis can be examined.

The above-mentioned model mice were divided into the four groups below, with 10 animals per group, at the age of 12 weeks and 3 days.
(First group) No treatment-STAM mice, pre-treatment control group (hereinafter, NT-STAM (Pre) group)
(Second group) No treatment-STAM mice group (hereinafter, NT-STAM group)
(Third group) 5% glucose treated group (hereinafter, Vehicle group)
(Fourth group) VA Liposome-siRNA Hsp47C treated group (hereinafter, VL-Hsp47C group)

### (2) Administration of administration solution

For the NT-STAM (Pre) group, after dividing into groups the mice were sacrificed before starting the treatment, and the clinical condition was checked. For the above-mentioned two groups other than the NT-STAM group, the corresponding administration solution below was administered by tail vein injection a total of 10 times every other day from the time of 12 weeks and 5 days old.
(Third group) As a solvent control, an administration solution (5% glucose or Vehicle) in which 0.75 mL/kg of body weight of nuclease-free water and 3.250 mL/kg of body weight of 5% glucose (Otsuka Pharmaceutical Co., Ltd.) were mixed was used.
(Fourth group) An administration solution (VA Liposome-siRNA Hsp47C or VL-Hsp47C) in which 75 nmol of VA, 75 nmol of liposome-constituting lipid, and 112.5 µg of siRNA were mixed per 100 µL of administration solution and final adjustment was carried out using 5% glucose was used.

The body weight at the date when administration was started was defined as the standard body weight, and 4 mL/kg of body weight of each administration solution was administered from the tail vein when the percentage body weight change at the date of administration was within 20% of the standard body weight. When it exceeded 20%, that body weight was thereafter defined as a new standard body weight, and the dose was reset.

### (3) Examination of improvement effect on renal fibrosis

On the 2^{nd} day after final administration was completed (15 weeks 4 days), the mice were sacrificed by taking blood from the heart under diethyl ether anesthesia, and the kidneys were removed.

The removed kidneys were fixed using a 4% paraformaldehyde-phosphoric acid buffer and embedded in paraffin, and thin section samples were prepared. In order to examine a therapeutic effect on renal fibrosis, Sirius red staining (fiber staining that specifically stains collagen red) was carried out, and an image was taken using a BZ-9000 all-in-one fluorescence microscope (Keyence Corporation) at 80×. Analysis was carried out by randomly taking images of 20 fields of view in the renal cortex region, and quantified using analysis software that came with the BZ-9000.

FIG. 1 shows a representative microscopic image of the glomerulus of the renal cortex region in each treated group. Sirius red staining is collagen-specific fiber staining, and a fibrosis site is stained with red to pink. From the microscopy results, no differences were seen between any of the groups with respect to thickening of the glomerular basement membrane, the degree of fibrosis in the mesangial region (stroma) and the surroundings of tubule cells was slight for the VL-Hsp47C treated group compared with the other three groups, and there was improvement in renal fibrosis (enlargement of mesangial region shown by arrow). Furthermore, 20 fields of view were randomly taken in the renal cortex region per individual mouse, and the proportion of the fibrotic region was calculated. From the results shown in FIG. 2, in the VL-Hsp47C treated group, the proportion of a Sirius red positive region was significantly lower than in the NT-STAM and Vehicle groups, and there was an improvement in renal fibrosis. Evaluation of statistically significant difference was carried out by use of the t-test.

From the result above, a tendency for the improvement of fibrosis in the VL-Hsp47C treated group was observed. Considering that siRNA basically acts within the cytoplasm, this result suggests that the retinoid functions as an agent targeting extracellular matrix-producing cells in the kidney, efficiently delivers a drug to same cells, and thus can suppress the progress of renal fibrosis.

### Example 3 Gene knockdown in extracellular matrix-producing cells in kidney by means of siRNA-containing VA-binding liposome (1) Isolation and collection of cells

Extracellular matrix-producing cells in the kidney having similar properties to those of hepatic stellate cells were isolated and collected as follows.

First, the five types of solutions below were prepared in advance. All of the solutions were stored at 4°C.
EGTA solution: 1.19 g of HEPES and 0.1 g of EGTA were added to 500 mL of HBSS (Invitrogen 14170) and mixed.
0.02% Collagenase solution: 1.19 g of HEPES, 0.235 g of CaCl₂ 2H₂O, and 0.1 g of Collagenase (Yakult YK-102) were added to 500 mL of HBSS (Invitrogen 24020) and mixed.
0.02% Collagenase + 0.1% Protease solution: 40 mg of Protease (Sigma P6911-1G) was added to 40 mL of 0.02% Collagenase and mixed. Hanks' solution: 0.05 g of MgSO₄ was added to 500 mL of HBSS (Invitrogen 24020) and mixed.
10% Nycodenz® solution (Axis-Shield Prod. No 1114542-1): 50 g of Nycodenz® was added to 500 mL of distilled water and mixed and dissolved.

A mouse (Male C57BL6/J, 20 to 30 g, 6 to 8 weeks old) was anesthetized, the abdominal area was shaved and an incision was made along the midline, and the kidneys were taken out. The kidneys thus taken out were washed with 30 mL of EGTA solution three times, thus removing blood. The kidneys were placed in 5 mL of EGTA solution, and finely cut using scissors. EGTA solution was added to the cut kidneys to make the total volume 30 mL, and centrifugation was carried out at 1300 rpm for 5 minutes. After the centrifugation, the supernatant was removed, 30 mL of 0.02% Collagenase + 0.1% Protease solution was added, and the mixture was transferred to an Erlenmeyer flask and stirred at 37°C for 20 minutes. The stirred solution was filtered using a Cell Strainer (Falcon 352360), and the filtrate was centrifuged at 1300 rpm for 5 minutes. The sediment was suspended in Hanks' solution, and 10% Nycodenz® solution was mixed with the Hanks' solution so as to give a final concentration of 8% Nycodenz®. The solution mixture was centrifuged at 1400×g for 20 minutes. The supernatant was collected and further centrifuged at 1300 rpm for 5 minutes. The supernatant was removed, the sample was resuspended in 10% FBS DMEM (Sigma D6546), and seeded on a T-75 flask (BD 353136).

### (2) Gene silencing experiment

### 1. Preparation of siRNA-containing VA-binding liposome

As the siRNA, one having the following sequence was used. Sequence name: Hsp47-C
5'-GGACAGGCCUGUACAACUA-dTdT-3'(sense, SEQ ID NO: 1)
5'-UAGUUGUACAGGCCUGUCC-dTdT-3'(antisense, SEQ ID NO: 2)

As solutions prior to mixing, 10 mM vitamin A (R7632, retinol, Sigma; hereinafter abbreviated to VA, dissolved in dimethyl sulfoxide), 1 mM Lipotrust SR (N301, Hokkaido System Science Co., Ltd. ; hereinafter also called a liposome or a liposome-constituting lipid, dissolved in nuclease-free water), and 10 mg/mL siRNA (Hsp47-C dissolved in nuclease-free water) were prepared. Subsequently, VA dissolved in dimethyl sulfoxide was added to the Lipotrust SR dissolved in nuclease-free water prepared above at a ratio of 1:1 (mol:mol), and the mixture was then stirred by means of a vortex for 15 seconds and allowed to stand at room temperature for 5 minutes in a light-shielded state, thus forming a complex. This complex was mixed with 10 mg/mL siRNA, thus giving VA Liposome-siRNA Hsp47C. When no VA was used, an equal amount of dimethyl sulfoxide was added instead of VA. Preparation was carried out such that when transfected to cells the final concentration of Lipotrust SR/VA was 7.7 µM and that of siRNA was 869 nM.

### 2. Transfection experiment

As cells for the experiment, the extracellular matrix-producing cells collected from mouse kidneys in the above-mentioned collection method were seeded in advance on a 6 well plate with 0.2 × 10⁵ cells/well and cultured in 10% FBS DMEM at 37°C for 2 days.

After 2 days of culturing, all of the 10% FBS DMEM of each well was removed prior to transfection, 900 µL of fresh 10% FBS DMEM was added, and culturing was carried out at 37°C for about 15 minutes.

100 µL of VA Liposome-siRNA Hsp47C or Liposome-siRNA Hsp47C prepared in '1.' above was added gently to each well and incubation was carried out at 37°C for 30 minutes, all of the culture supernatant of each well was then removed, 2 mL of fresh 10% FBS DMEM was added, and culturing was carried out at 37°C for 2 days. After 2 days RNA was isolated from the cells using an RNeasy Mini Kit(QIAGEN 74104), and RT-PCR was carried out by means of a High Capacity RNA-to-cDNA Master Mix (Applied Biosystems 4390779), thus giving cDNA. Quantitative PCR was carried out by means of a Power SYBR® Green PCR Master Mix (Applied Biosystems 4368708) using the cDNA thus obtained, and the effect in gene silencing was examined. The results are shown in FIG. 3.

The above results show that the siRNA for the HSP47 gene used in Example 2 suppresses the expression of the HSP47 gene in extracellular matrix-producing cells of the kidney, suggesting that the siRNA contained in the treatment agent of the present invention is specifically incorporated into the extracellular matrix-producing cells of the kidney and suppresses the expression of the target gene within the cells, thus suppressing the progress of renal fibrosis.

## Claims

1. A pharmaceutical composition for use in treating diabetic nephritis, the composition comprising a carrier comprising a retinoid that promotes the delivery of a substance to extracellular matrix-producing cells in the kidney and a drug for controlling the activity or growth of extracellular matrix-producing cells in the kidney, wherein the carrier has the form of a liposome, and the molar ratio of retinoid and lipid contained in the liposome is 8:1 to 1:4.

2. The pharmaceutical composition for use according to Claim 1, wherein the retinoid comprises retinol.

3. The pharmaceutical composition for use according to any one of Claim 1 or 2, wherein the drug for controlling the activity or growth of extracellular matrix-producing cells in the kidney is selected from the group consisting of an inhibitor of PAI-1 activity or production, a cell activity inhibitor, a growth inhibitor, an apoptosis inducer, and an RNAi molecule, ribozyme, antisense nucleic acid, or DNA/RNA chimeric polynucleotide that target at least one of the extracellular matrix constituent molecules or molecules involved in the production or secretion of the extracellular matrix constituent molecules or a vector expressing same.

4. The pharmaceutical composition for use according to any one of Claim 1 or 2, wherein the drug for controlling the activity or growth of extracellular matrix-producing cells is an HSP47 inhibitor.

5. The pharmaceutical composition for use according to any one of Claims 1 to 4, wherein the drug and the carrier are mixed at a place of medical treatment or in its vicinity.

## Patentansprüche

1. Pharmazeutische Zusammensetzung zur Verwendung bei der Behandlung von diabetischer Nephritis, wobei die Zusammensetzung einen Träger umfasst, umfassend Retinoid, das die Abgabe einer Substanz an extrazelluläre Matrix produzierende Zellen in der Niere fördert und ein Arzneimittel zur Steuerung der Aktivität oder des Wachstums von extrazelluläre Matrix produzierenden Zellen in der Niere, wobei der Träger die Form eines Liposoms aufweist und das Molverhältnis von Retinoid und Lipid, das im Liposom enthalten ist, 8:1 bis 1:4 beträgt.

2. Pharmazeutische Zusammensetzung zur Verwendung gemäß Anspruch 1, wobei das Retinoid Retinol umfasst.

3. Pharmazeutische Zusammensetzung zur Verwendung gemäß einem der Ansprüche 1 oder 2, wobei das Arzneimittel zur Steuerung der Aktivität oder des Wachstums von extrazelluläre Matrix produzierenden Zellen in der Niere, ausgewählt ist aus der Gruppe bestehend aus einem Inhibitor der PAI-1-Aktivität oder -Produktion, einem Zellaktivitätsinhibitor, einem Wachstumsinhibitor, einem Apoptoseinduktor und einem RNAi-Molekül, Ribozym, Antisense-Nukleinsäure oder chimären DNA/RNA-Polynukleotiden, die auf mindestens eines der Moleküle, die die extrazelluläre Matrix bilden oder der Moleküle, die an der Produktion oder Sekretion der Moleküle, die die extrazelluläre Matrix bilden, beteiligt sind, oder einen Vektor, der diese exprimiert, zielen.

4. Pharmazeutische Zusammensetzung zur Verwendung gemäß einem der Ansprüche 1 oder 2, wobei das Arzneimittel zur Steuerung der Aktivität oder des Wachstums von extrazelluläre Matrix produzierenden Zellen ein HSP47-Inhibitor ist.

5. Pharmazeutische Zusammensetzung zur Verwendung gemäß einem der Ansprüche 1 bis 4, wobei das Arzneimittel und der Träger am Ort der medizinischen Behandlung oder in dessen Nähe vermischt werden.

## Revendications

1. Composition pharmaceutique pour une utilisation dans le traitement de néphropathie diabétique, la composition comprenant un véhicule comprenant un rétinoïde qui favorise l'administration d'une substance à des cellules productrices de matrice extracellulaire dans le rein, et un médicament pour contrôler l'activité ou la croissance de cellules productrices de matrice extracellulaire dans le rein dans laquelle le véhicule a la forme d'un liposome, et le rapport molaire du rétinoïde et du lipide contenu dans le liposome est de 8:1 à 1:4.

2. Composition pharmaceutique pour une utilisation selon la revendication 1, dans laquelle le rétinoïde comprend du rétinol.

3. Composition pharmaceutique pour une utilisation selon l'une quelconque des revendications 1 ou 2, dans laquelle le médicament pour contrôler l'activité ou la croissance de cellules productrices de matrice extracellulaire dans le rein est choisi dans le groupe constitué par un inhibiteur d'activité ou de production de PAI-1, un inhibiteur d'activité cellulaire, un inhibiteur de croissance, un inducteur d'apoptose, et une molécule d'ARNi, un ribozyme, un acide nucléique antisens, ou un polynucléotide chimérique ADN/ARN qui ciblent au moins une des molécules constitutives de la matrice extracellulaire ou molécules impliquées dans la production ou la sécrétion des molécules constitutives de la matrice extracellulaire ou un vecteur les exprimant.

4. Composition pharmaceutique pour une utilisation selon l'une quelconque des revendications 1 ou 2, dans laquelle le médicament pour contrôler l'activité ou la croissance de cellules productrices de matrice extracellulaire est un inhibiteur de HSP47.

5. Composition pharmaceutique pour une utilisation selon l'une quelconque des revendications 1 à 4, dans laquelle le médicament et le véhicule sont mélangés sur le lieu du traitement médical ou à sa proximité.
